# EUROPEAN PATENT APPLICATION

(11) **EP 3 546 019 A1**
(43) Date of publication of application: **02.10.2019**
(21) Application number: 19000140.4
(22) Date of filing: 21.03.2019
(51) Int. Cl.: A61N 2/02, A61N 2/00, A61N 7/00, A61N 5/06

(54) **IMPROVED EFFICACY MAGNETOTHERAPEUTIC DEVICE**

(30) Priority: 27.03.2018 IT 201800004007
(71) Applicant: Alberton, Gabriele, 23880 Casatenovo (LC) (IT)
(72) Inventor: Alberton, Gabriele, 23880 Casatenovo (LC) (IT)
(74) Representative: Mocchetti, Ilio G.

(57) **Abstract**

The invention relates to a device capable of subjecting a living biological tissue to a simultaneous magnetotherapeutic and phototherapeutic treatment. Namely, the device the object of the invention includes a first component suitable for emitting a low-frequency time-varying magnetic field, a second component suitable for emitting a high-frequency time-varying magnetic field and a third component suitable for emitting electromagnetic waves having a wavelength between 10 nm and 1 mm. The device the object of the invention may further comprise a fourth component suitable for emitting ultrasonic waves and a fifth component suitable for emitting heat.

## Description

### Field of application of the invention

The present invention is applied in the field of medical devices usually defined as "magnetotherapeutic" devices, i.e. devices designed to stimulate living biological tissues for therapeutic purposes by irradiating the latter with static or time-varying magnetic fields, preferably of the pulsed or alternating type. Incidentally, a static magnetic field is a magnetic field which can be schematized in an intensity-time diagram by means of a straight line. A pulsed magnetic field is a magnetic field which can be schematized in an intensity-time diagram by means of a sequence of half waves of the same sign. An alternating magnetic field is a magnetic field which can be schematized in an intensity-time diagram by means of a sequence of half waves of alternating opposite sign (i.e. the schematization of which in a diagram of the aforesaid type has a wavy trend repeatedly intersecting the time axis).

The invention in particular relates to magnetotherapeutic devices emitting time-varying magnetic fields. For convenience of disclosure, the term "magnetotherapeutic device" in the present description below exclusively refers to a device of the aforesaid type, i.e. to a device capable of irradiating a living biological tissue with a time-varying magnetic field.

Incidentally, in order for the exposure of a biological tissue to a time-varying magnetic field to have a therapeutic effect, the frequency of the magnetic field is to be in the order of Hertz or Megahertz. In light of this, magnetotherapeutic devices usually are designed so as to emit magnetic fields at the frequencies indicated above. Namely, a magnetotherapeutic device is defined "low frequency" if it generates a magnetic field having a frequency between 5 Hz and 200 Hz, and "high frequency" if it generates a magnetic field having a frequency between 18 MHz and 900 MHz.

### Overview of the prior art and outline of the technical problem

The current magnetotherapeutic devices are devices capable of exclusively performing low- and/or high-frequency magnetotherapeutic treatments. Studies conducted by the Applicant have revealed that the efficacy of a magnetotherapeutic treatment is greater if the latter is simultaneously combined with a phototherapeutic treatment, i.e. a therapeutic treatment consisting in stimulating a living biological tissue by means of irradiating the same with electromagnetic waves having a frequency almost between 30 Phz and 300 GHz (i.e. a wavelength between 10 nm and 1 mm). With reference to an electromagnetic spectrum, the electromagnetic waves with which a living biological tissue is irradiated within the scope of a phototherapeutic treatment generally are visible or ultraviolet infrared radiations.

In other words, the studies conducted by the Applicant have revealed that the efficacy of a magnetotherapeutic treatment is greater if a living biological tissue not only is irradiated with a low- and/or high-frequency magnetic field, but also simultaneously with electromagnetic waves having a wavelength between 10 nm and 1 mm.

The Applicant is not aware of any medical device capable of simultaneously performing a magnetotherapeutic treatment and a phototherapeutic treatment.

### Objects of the invention

It is the object of the present invention to overcome the aforesaid drawbacks by indicating a device capable of stimulating a living biological tissue by simultaneously irradiating it both with a low-frequency and/or a high-frequency time-varying magnetic field, and with electromagnetic waves having a wavelength preferably between 10 nm and 1 mm.

### Summary of the invention

The object of the present invention is a device, comprising:
- first means for emitting a low-frequency time-varying magnetic field (i.e. at a frequency between 5 Hz and 200 Hz);
   and/or
- second means for emitting a high-frequency time-varying magnetic field (i.e. at a frequency between 18 Hz and 900 Hz);
in which, according to the invention, said device further comprises:
- third means for emitting electromagnetic waves having a wavelength between 10 nm and 1 mm;
- control means of said first, second and third emitter means, said control means being suitable for activating said first and/or second emitter means simultaneously to said third emitter means;
said first, second and third emitter means being arranged in such a way that the magnetic field emitted by said first and/or second emitter means, when activated, at least partly overlaps the electromagnetic waves emitted by said third emitter means, when activated.

Other innovative features of the present invention are illustrated in the following description and are referred to in the dependent claims.

Advantageously, the device the object of the invention is capable of stimulating, for therapeutic purposes, a living biological tissue by simultaneously irradiating the latter with a low- and/or a high-frequency time-varying magnetic field, and with electromagnetic waves having a wavelength preferably between 10 nm and 1 mm. The device the object of the invention is therefore capable of subjecting a living biological tissue both to a simultaneous magnetotherapeutic and phototherapeutic treatment. As mentioned above, the combination under conditions of simultaneity, of a phototherapeutic treatment with a magnetotherapeutic treatment significantly increases the efficacy of the latter. The therapeutic efficacy of the device the object of the invention also is greater if the control means are suitable for activating said third emitter means in such a way that the latter emit said electromagnetic waves in pulsed mode. This means that the emission of said electromagnetic waves can be schematized in an intensity-time diagram by means of a sequence of half waves of the same sign. Finally, the therapeutic efficacy of the device the object of the invention is even greater if, in addition to being suitable for activating said third emitter means in such a way that the latter emit said electromagnetic waves in pulsed mode, the control means are suitable for activating said third emitter means so as to synchronize the frequency with which the pulse of said electromagnetic waves occurs to the frequency of the time-varying magnetic field emitted by said first or second emitter means.

According to one aspect of the invention, said third emitter means comprise a plurality of substantially punctiform sources of said electromagnetic waves arranged along a closed line,
said first and/or second emitter means being arranged in such a way that the magnetic field emitted by said first and/or second emitter means, when activated, at least partly crosses a surface delimited by said closed line. Advantageously, according to this aspect of the invention, the first, second and third emitter means are arranged so as to optimize the space occupied by them. According to another aspect of the invention, the device comprises fourth emitter means of ultrasonic waves (i.e. mechanical waves which propagate into the air and have a frequency not lower than 20 kHz),
said control means acting as control means also of said fourth emitter means and being suitable for activating said fourth emitter means simultaneously to said first and/or second emitter means and to said third emitter means,
said fourth emitter means being arranged in such a way that the ultrasonic waves emitted by said fourth emitter means, when activated, at least partly overlap the magnetic field emitted by said first and/or second emitter means, when activated, and/or the electromagnetic waves emitted by said third emitter means, when activated.

Advantageously, the simultaneous stimulation of a living biological tissue by means of magnetotherapy, phototherapy and ultrasound therapy further increases the therapeutic efficacy of the device the object of the invention.

The therapeutic efficacy also is greater if the control means are suitable for activating said fourth emitter means in such a way that the latter emit said ultrasonic waves in pulsed mode. This means that the emission of said ultrasonic waves can be schematized in an intensity-time diagram by means of a sequence of half waves of the same sign.

The therapeutic efficacy of the device the object of the invention finally is even greater if, in addition to being suitable for activating said fourth emitter means in such a way that the latter emit said electromagnetic waves in pulsed mode, the control means are suitable for activating said fourth emitter means so as to synchronize the frequency with which the pulse of said ultrasonic waves occurs to the frequency of the time-varying magnetic field emitted by said first or second emitter means.

According to another aspect of the invention, the device comprises heating means suitable for yielding heat by heat radiation,
said control means acting as control means also of said heating means and being suitable for activating said heating means simultaneously to said first and/or second emitter means and to said third emitter means,
said heating means being arranged in such a way that the electromagnetic waves with which said heat radiation takes place when said heating means are activated, at least partly overlap the magnetic field emitted by said first and/or second emitter means, when activated, and/or the electromagnetic waves emitted by said third emitter means, when activated.

Advantageously, the heat further increases the therapeutic efficacy of the device the object of the invention.

According to another aspect of the invention, said first and/or second emitter means themselves serve as said heating means.

According to this aspect of the invention, the first and/or second emitter means themselves advantageously emit heat in conjunction with a low- or high-frequency magnetic field.

According to another aspect of the invention, said third emitter means themselves serve as said heating means.

According to this aspect of the invention, the third emitter means themselves advantageously emit heat in conjunction with electromagnetic waves having a wavelength preferably between 10 nm and 1 mm.

### Brief description of the drawings

Further objects and advantages of the present invention will become apparent from the detailed description provided below of example embodiments thereof and from the accompanying drawings merely given by way of a non-limiting example, in which:
- **figure 1** diagrammatically shows a view of a device according to the present invention;
- **figure 2** diagrammatically shows an embodiment of the device in figure 1.

### Detailed description of some preferred embodiments of the invention

In the continuation of the present description, a figure may also be shown with reference to elements not expressly indicated in that figure but in other figures. The scale and proportions of the different elements depicted do not necessarily correspond to the actual ones.

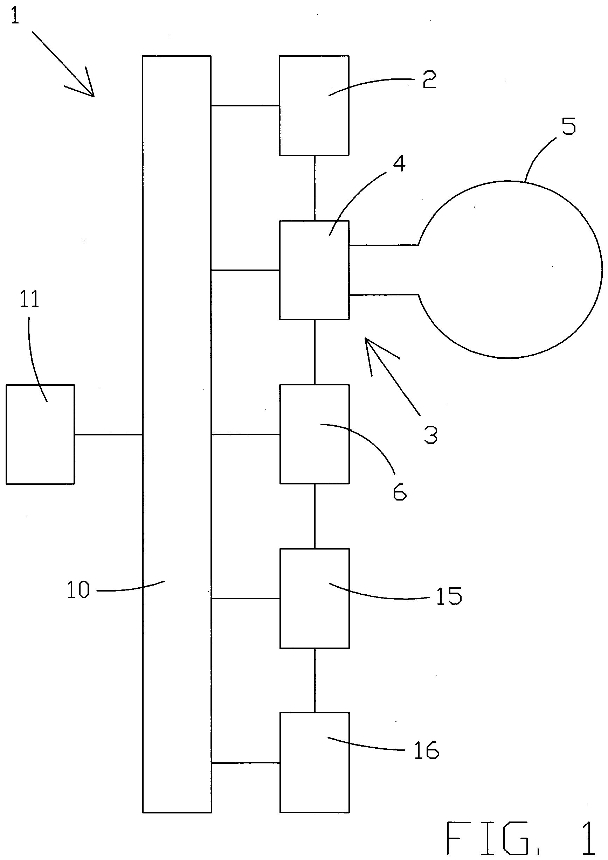

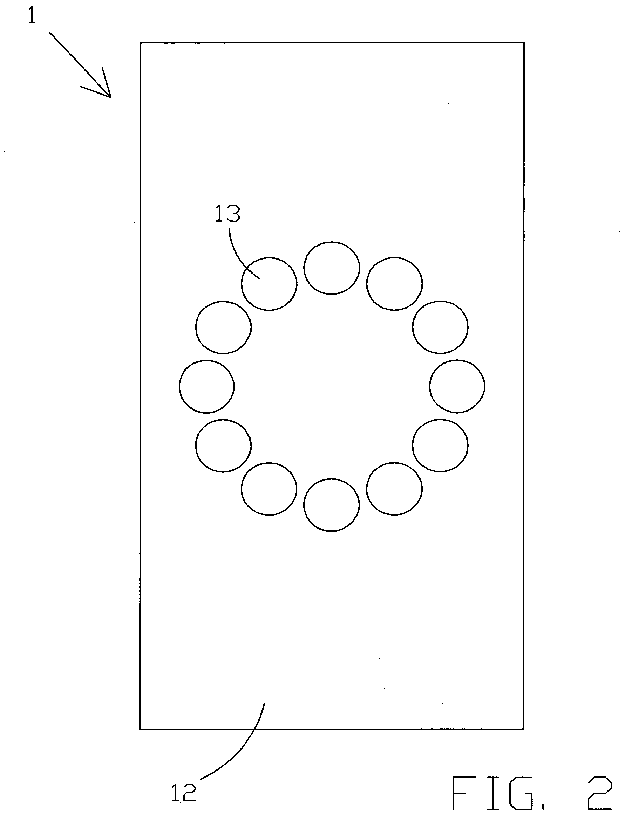

**Figures 1** **and** **2** show a device 1, the object of the invention, capable of subjecting a living biological tissue to a low-frequency (i.e. at a frequency between 5 Hz and 200 Hz) or to a high-frequency (i.e. at a frequency between 18 MHz and 900 MHz) magnetotherapeutic treatment and simultaneously to a phototherapeutic treatment.

For this purpose, device 1 comprises a component 2 suitable for emitting a low-frequency time-varying magnetic field. Component 2, indicated above with the term "first emitter means", includes by way of example, an oscillator capable of generating an electrical signal at a frequency preferably between 5 Hz and 200 Hz. The oscillator is connected to a solenoid including a plurality of coils. When the electrical signal generated by the oscillator travels the solenoid, the latter emits an electromagnetic field which frequency is in the same order of magnitude as the one of the aforesaid electrical signal.

Alternatively to the aforesaid oscillator connected to a solenoid, component 2 could comprise an electric motor, preferably of the step-step type, suitable for rotating a shaft about a longitudinal axis thereof. A rotor is rotationally connected to the motor by means of the aforesaid shaft. Due to the rotational type connection between the shaft and the rotor, the motor is suitable for rotating the rotor about the aforesaid axis. The rotor preferably is discoidal and preferably is centrally connected to the shaft. More preferably, the rotor is connected to the shaft orthogonally to the latter. The rotor includes a plurality of permanent magnets. The latter preferably are equal to one another and preferably are shaped in such a way that each magnet can be schematized by means of a magnetic dipole. As is known, magnetic dipoles can be depicted by means of a vector which extends from the positive (or "north") pole of the dipole to the negative (or "south") pole thereof. The magnets preferably are integrally snappingly connected to the rotor. Namely, the magnets preferably are accommodated in respective seats made radially in the rotor, preferably one adjacent to the other, from a side wall thereof. Said housings ensure the magnets are interlocked in the rotor. The magnets are accommodated in said seats preferably in such a way that the vectors of the magnetic dipoles schematizing them are arranged orthogonally to the aforesaid rotation axis, i.e. radially with respect to the rotor. The motor is suitable for rotating the magnets about the aforesaid axis due to the integral connection between the magnets and the rotor and the rotational connection between the shaft and the rotor. The rotation of the magnets with respect to a biological tissue to be subjected to a magnetotherapeutic treatment ensures said tissue is irradiated with a low-frequency time-varying magnetic field. Alternatively to being connected to the rotor in such a way that the vectors of the magnetic dipoles are arranged orthogonal to the rotation axis of the rotor, the magnets may be connected to the rotor in such a way that the vectors of the magnetic dipoles are arranged parallel to the rotation axis of the rotor. Greater details concerning the embodiment of the above-described component 2 can be obtained in Italian Patent Application for industrial invention no. 102017000125370.

Additionally or alternatively to component 2, device 1 comprises a component 3 suitable for emitting a high-frequency time-varying magnetic field. Component 3, indicated above with the term "second emitter means", includes by way of example, an oscillator 4 capable of generating an electrical signal at a frequency preferably between 18 MHz and 900 MHz, and more preferably of 27 MHz. Oscillator 4 is connected to an antenna 5 including at least one coil which, when travelled by the electric current generated by oscillator 4, emits electromagnetic waves. Moreover, oscillator 4 preferably is connected to a modulator for generating an electrical signal that modulates in amplitude the signal generated by oscillator 4. Said modulation preferably takes place in such a way that the electromagnetic field transmitted by antenna 5 is of the square wave pulsed type. The modulation frequency preferably is between 200 Hz and 15 KHz.

In addition to one or both the components 2 and 3, device 1 comprises a third component 6 suitable for emitting electromagnetic waves having a wavelength between 10 nm and 1 mm. Component 6, indicated above with the term "third emitter means", by way of example includes one or more LEDs (i.e. light emitting diodes) or a laser (i.e. an emitter of a coherent electromagnetic wave beam) or a light source of incandescent or gas-discharge type.

Device 1 also comprises a control unit 10 connected to the aforesaid components 2 and/or 3 and 6. Unit 10 is suitable both for activating the components 2 and/or 3 so the latter emit a low and/or high-frequency time-varying magnetic field, respectively, and for activating component 6 so the latter emits electromagnetic waves having a wavelength between 10 nm and 1 mm. Namely, unit 10 is suitable for activating the components 2 and/or 3 simultaneously to component 6. The control unit 10 is also connected to an electric accumulator 11 for supplying the components 2 and/or 3 and 6 of unit 10 itself.

The components 2 and/or 3 and 6 are arranged in such a way that the magnetic field emitted by the components 2 and/or 3 (when activated) at least partly overlap, externally to device 1, the electromagnetic waves emitted by component 6 (when activated). Given that unit 10 is suitable for activating the components 2 and/or 3 simultaneously to component 6, device 1 allows a living biological tissue to be stimulated by simultaneously irradiating the latter with a low- and/or a high-frequency time-varying magnetic field and with electromagnetic waves having a wavelength preferably between 10 nm and 1 mm. In particular, in order to subject a living biological tissue to a magnetotherapeutic and simultaneously phototherapeutic treatment, it is sufficient to activate the components 2 and/or 3 and 6 by means of unit 10 and to orient device 1 in such a way that the portion of low- and/or high-frequency magnetic field overlapping the electromagnetic waves emitted by component 6 hits the aforesaid tissue.

The components 2 and/or 3 and 6, unit 10 and accumulator 11 preferably are enclosed in a casing including a wall 12 at which device 1 may be put into contact with a part of human body. The components 2 and/or 3 preferably are arranged in such a way that when wall 12 is in contact with a part of human body, the magnetic field emitted by the components 2 and/or 3 (when activated) crosses wall 12, thus irradiating the aforesaid part of human body. Similarly, component 6 preferably is arranged in such a way that when wall 12 is in contact with a part of human body, the electromagnetic waves emitted by component 6 (when activated) irradiate the aforesaid part of human body. In light of what is said above, the components 2 and/or 3 and 6 are also arranged in such a way that the magnetic field emitted by the components 2 and/or 3 (when activated) at least partly overlap, externally to device 1 (i.e. beyond wall 12), the electromagnetic waves emitted by component 6 (when activated).

As mentioned above, the combination under conditions of simultaneity, of a phototherapeutic treatment with a magnetotherapeutic treatment significantly increases the efficacy of the latter. In light of the above, component 6 preferably can be activated by unit 10 so that the electromagnetic waves are emitted over time in continuous mode or in pulsed mode. The latter mode is more preferable since the therapeutic efficacy of device 1 is greater in this case. Even more preferably, component 6 can be activated by unit 10 so that the electromagnetic waves are emitted over time not only in pulsed mode, but also so as to synchronize the frequency with which the pulse of the electromagnetic waves occurs to the frequency of the magnetic field emitted by the components 2 and/or 3, when activated.

In a preferred embodiment of device 1 (diagrammatically shown in figure 2), component 6 comprises a plurality of substantially punctiform sources 13 arranged along a closed line. The sources 13 may consist of LEDs suitable for emitting electromagnetic waves having a wavelength between 10 nm and 1 mm. Said closed line preferably consists of a circumference along which the sources 13 preferably are equally spaced apart from one another. The sources 13 preferably are accommodated in respective seats obtained in wall 12 and are arranged so as to emit (when activated) electromagnetic waves towards the outside of device 1. The components 2 and/or 3 preferably are arranged (inside device 1) in such a way that the magnetic field emitted by them (when activated) at least partly crosses a surface delimited by the aforesaid closed line. For this purpose, the components 2 and/or 3 advantageously may be accommodated in a portion of space enclosed by a cylindrical surface having as base the circular crown defined by the sources 13.

In addition to the components 2 and/or 3 and 6, device 1 could comprise a fourth component 15 (indicated above with the term "fourth emitter means") suitable for emitting ultrasonic waves. Unit 10 is also connected to component 15 and is suitable for activating the latter so that it emits ultrasonic waves. Namely, unit 10 is suitable for activating component 15 simultaneously to the components 2 and/or 3 and to component 6. Component 15 is supplied by means of accumulator 11.

Component 15 is arranged in such a way that the ultrasonic waves emitted by it (when activated) at least partly overlap, externally to device 1, the magnetic field emitted by the components 2 and/or 3 (when activated) and/or the electromagnetic waves emitted by component 6 (when activated). Given that unit 10 is suitable for activating component 15 simultaneously to the components 2 and/or 3 and to component 6, device 1 allows a living biological tissue to be stimulated by simultaneously irradiating the latter with a low- and/or a high-frequency time-varying magnetic field, with electromagnetic waves having a wavelength preferably between 10 nm and 1 mm, and with ultrasonic waves. In particular, in order to subject a living biological tissue to a magnetotherapeutic and simultaneously phototherapeutic and ultrasound therapeutic treatment, it is sufficient to activate the components 2 and/or 3, 6 and 15 by means of unit 10 and to orient device 1 in such a way that the portion of low- and/or high-frequency magnetic field overlapping the electromagnetic waves emitted by component 6 and the ultrasonic waves emitted by component 15 hits the aforesaid tissue.

Like the components 2 and/or 3 and 6 of unit 10 and of accumulator 11, also component 15 preferably is enclosed in the casing including wall 12 (at which device 1 may be put into contact with a part of human body). Component 15 preferably is arranged in such a way that when wall 12 is in contact with a part of human body, the ultrasonic waves emitted by component 15 (when activated) irradiate the aforesaid part of human body. In light of what is said above, the components 2 and/or 3, 6 and 15 are also arranged in such a way that the ultrasonic waves emitted by component 15 (when activated) at least partly overlap, externally to device 1 (i.e. beyond wall 12), the magnetic field emitted by the components 2 and/or 3 (when activated) and the electromagnetic waves emitted by component 6 (when activated).

As mentioned above, the combination under conditions of simultaneity, of a magnetotherapeutic and phototherapeutic treatment with an ultrasound therapeutic treatment significantly increases the therapeutic efficacy of device 1. In light of the above, component 15 preferably can be activated by unit 10 so that the ultrasonic waves are emitted over time in continuous mode or in pulsed mode. The latter mode is more preferable since the therapeutic efficacy of device 1 is greater in this case. Even more preferably, component 15 can be activated by unit 10 so that the ultrasonic waves are emitted over time not only in pulsed mode, but also so as to synchronize the frequency with which the pulse of the ultrasonic waves occurs to the frequency of the magnetic field emitted by the components 2 and/or 3, when activated.

In addition to the components 2 and/or 3, 6 and possibly 15, device 1 could comprise a fifth component 16 (indicated above with the term "heating means") suitable for yielding heat by heat radiation. Unit 10 is also connected to component 16 and is suitable for activating the latter so that it yields heat. Namely, unit 10 is suitable for activating component 16 simultaneously to the components 2 and/or 3, 6 and, if present, 15. Component 16 is supplied by means of accumulator 11.

Component 16 is arranged in such a way that the electromagnetic waves (with which the heat radiation takes place) emitted by it (when activated) at least partly overlap, externally to device 1, the magnetic field emitted by the components 2 and/or 3 (when activated) and/or the electromagnetic waves emitted by component 6 (when activated) and/or the ultrasonic waves emitted by component 15 (if present and when activated). Given that unit 10 is suitable for activating component 16 simultaneously to the components 2 and/or 3, 6 and if present, 15, device 1 allows a living biological tissue to be stimulated by heating it and simultaneously irradiating it with a low- and/or a high-frequency time-varying magnetic field, with electromagnetic waves having a wavelength preferably between 10 nm and 1 mm, and if present, component 15, with ultrasonic waves. In particular, in order to heat a living biological tissue by simultaneously subjecting it to a magnetotherapeutic, phototherapeutic and, if component 15 is present, ultrasound therapeutic treatment, it is sufficient to activate the components 2 and/or 3, 6, 16 and possibly 15 by means of unit 10 and to orient device 1 in such a way that the portion of low- and/or high-frequency magnetic field overlapping the electromagnetic waves emitted by component 6, the electromagnetic waves emitted by component 16 and the ultrasonic waves emitted by component 15 (if present), hits the aforesaid tissue. Like the components 2 and/or 3, 6 and 15 of unit 10 and of accumulator 11, also component 16 preferably is enclosed in the casing including wall 12 (at which device 1 may be put into contact with a part of human body). Component 16 preferably is arranged in such a way that when wall 12 is in contact with a part of human body, the electromagnetic waves emitted by component 16 (when activated) irradiate, heat, the aforesaid part of human body. In light of what is said above, the components 2 and/or 3, 6, 16 and if present 15, are also arranged in such a way that the electromagnetic waves emitted by component 16 (when activated) at least partly overlap, externally to device 1 (i.e. beyond wall 12), the magnetic field emitted by the components 2 and/or 3 (when activated), the electromagnetic waves emitted by component 6 (when activated) and the ultrasonic waves emitted by component 15 (if present and when activated).

As mentioned above, the heating of the biological tissue subjected, under conditions of simultaneity, to a magnetotherapeutic, phototherapeutic and possibly ultrasound therapeutic treatment, significantly increases the therapeutic efficacy of device 1.

According to an alternative embodiment of device 1, the same components 2 and/or 3 and/or 6 are suitable for yielding heat by heat radiation, and therefore for serving as component 16.

Incidentally, not only can the components 2 and/or 3, 6 and if present 15 and 16, be activated by unit 10 simultaneously, but they can also be activated individually. A user of device 1 may thus decide to simultaneously activate all the aforesaid components or only one or some of them. A user of device 1 may also adjust the intensity of the waves emitted by the aforesaid components.

On the basis of the description provided for a preferred example embodiment, it is obvious that certain changes can be made by those skilled in the art without departing from the scope of the invention as defined by the following claims.

## Claims

1. A device (1) comprising:
• first means (2) for emitting a low-frequency time-varying magnetic field;
and/or
• second means (3, 4, 5) for emitting a high-frequency time-varying magnetic field;
said device (1) being **characterized in that** it further comprises:
• third means (6) for emitting electromagnetic waves having a wavelength between 10 nm and 1 mm;
• control means (10) of said first, second and third emitter means (2, 3, 4, 5, 6), said control means (10) being suitable for activating said first and/or second emitter means (2, 3, 4, 5) simultaneously to said third emitter means (6);
said first, second and third emitter means (2, 3, 4, 5, 6) being arranged in such a way that the magnetic field emitted by said first and/or second emitter means (2, 3, 4, 5), when activated, at least partly overlaps the electromagnetic waves emitted by said third emitter means (6), when activated.

2. A device (1) according to claim 1, **characterized in that** said control means (10) are suitable for activating said third emitter means (6) in such a way that the latter emit said electromagnetic waves in pulsed mode.

3. A device (1) according to claim 2, **characterized in that** said control means (10) are suitable for activating said third emitter means (6) so as to synchronize the frequency with which the pulse of said electromagnetic waves occurs with the frequency of the magnetic field emitted by said first or second emitter means (2, 3, 4, 5).

4. A device (1) according to one of the preceding claims, **characterized in that** said third emitter means (6) comprise a plurality of substantially punctiform sources (13) of said electromagnetic waves arranged along a closed line, said first and/or second emitter means (2, 3, 4, 5) being arranged in such a way that the magnetic field emitted by said first and/or second emitter means (2, 3, 4, 5), when activated, at least partly crosses a surface delimited by said closed line.

5. A device (1) according to one of the preceding claims, **characterized in that** it comprises fourth means (15) for emitting ultrasonic waves,
said control means (10) acting as control means also of said fourth emitter means (15), and being suitable for activating said fourth emitter means (15) simultaneously to said first and/or second emitter means (2, 3, 4, 5) and to said third emitter means (6),
said fourth emitter means (15) being arranged in such a way that the ultrasonic waves emitted by said fourth emitter means (15), when activated, at least partly overlap the magnetic field emitted by said first and/or second emitter means (2, 3, 4, 5), when activated, and/or the electromagnetic waves emitted by said third emitter means (6), when activated.

6. A device (1) according to claim 5, **characterized in that** said control means (10) are suitable for activating said fourth emitter means (15) in such a way that the latter emit said ultrasonic waves in pulsed mode.

7. A device (1) according to claim 6, **characterized in that** said control means (10) are suitable for activating said fourth emitter means (15) so as to synchronize the frequency with which the pulse of said ultrasonic waves occurs with the frequency of the magnetic field emitted by said first or second emitter means (2, 3, 4, 5).

8. A device (1) according to one of the preceding claims, **characterized in that** it comprises heating means (16) suitable for transferring heat by heat radiation,
said control means (10) acting as control means also of said heating means (16), and being suitable for activating said heating means (16) simultaneously to said first and/or second emitter means (2, 3, 4, 5) and to said third emitter means (6),
said heating means (16) being arranged in such a way that the electromagnetic waves with which said heat radiation takes place when said heating means (16) are activated, at least partly overlap the magnetic field emitted by said first and/or second emitter means (2, 3, 4, 5), when activated, and/or the electromagnetic waves emitted by said third emitter means (6), when activated.

9. A device according to claim 8, **characterized in that** said first and/or second emitter means serve as said heating means.

10. A device according to claim 8 or 9, **characterized in that** said third emitter means serve as said heating means.
